# EUROPEAN PATENT APPLICATION

(11) **EP 4 411 346 A1**
(43) Date of publication of application: **07.08.2024**
(21) Application number: 22875713.4
(22) Date of filing: 02.09.2022
(51) Int. Cl.: G01N 21/61, G01N 21/3504

(54) **OPTICAL GAS SENSOR DEVICE**

(30) Priority: 29.09.2021 JP 2021159087
(71) Applicant: MITSUMI ELECTRIC CO., LTD., Tama-Shi, Tokyo 206-8567 (JP)
(72) Inventor: NAKAMURA Akira, Tama-shi, Tokyo 206-8567 (JP)
(74) Representative: Epping - Hermann - Fischer
(86) International application number: PCT/JP2022/033050
(87) International publication number: WO 2023/053841

(57) **Abstract**

The objective of the present invention is to reduce the size and power consumption of a non dispersive infrared (NDIR) optical gas sensor device, and to increase the service life thereof. An optical gas sensor device 100 comprises: a board 6; a light source 2 which is mounted on a flat surface of the board 6, has a light emitting surface that faces in the same direction as the flat surface of the board 6, and emits infrared light from the light emitting surface; an optical filter 3 which, from among the emitted infrared light, transmits infrared light having a wavelength corresponding to an absorption wavelength of a gas G to be detected, and emits the same into the gas G to be detected; a light receiving unit 4 for detecting infrared light that is incident thereon via the gas G to be detected; a cover 1 which is provided on the board 6 so as to cover the light source 2 and the light receiving unit 4, has an inner surface which reflects infrared light that has passed through the optical filter 3, and is provided in such a way that at least a portion of the reflected light reaches the light receiving unit 4; and a gas introduction port 11 for introducing the gas G to be detected into the interior of the cover 1.

## Description

### TECHNICAL FIELD

The present invention relates to an optical gas sensor device.

### BACKGROUND ART

Conventionally, gas sensors using a non-dispersive infrared (NDIR) absorption method are known. NDIR gas sensors take advantage of the property that many gases each absorb a specific infrared wavelength. When infrared rays are emitted to the gas as the detection target, the NDIR gas sensor detects which wavelength is absorbed and how much. With this, the concentration in the gas as the detection target is measured.

For example, a well-known gas sensor includes an infrared light emitter and an infrared light receiver, and a reflector arranged on the optical path of the light emitter and the light receiver, and detects the concentration of the gas to be detected between the light emitter, the reflector and the light receiver (see Patent Document 1).

Also, an infrared gas analyzer is known that detects the concentration of the gas component to be measured in a sample gas. The analyzer includes an infrared light source, a photoconductive infrared detection sensor, a measurement cell supplied with sample gas, a reference cell filled with inert gas, and a rotating filter chopper equipped with an optical filter for a specific gas. The infrared rays from the infrared light source are filtered by the rotating filter chopper through the sample gas of the measurement cell and the inert gas of the reference cell, and detected by the photoconductive infrared detection sensor (see Patent Document 2).

### CITATION LIST

### Patent Literature

Patent Document 1: Patent No. 6626281
Patent Document 2: Japanese Unexamined Patent Application Publication No. H8-75642

### SUMMARY OF INVENTION

### Technical Problem

Although the configurations of the above-mentioned conventional gas sensor and infrared gas analyzer as NDIR optical gas sensor devices are known, there is a demand for a device with a smaller size, lower power consumption, and a longer service life.

An object of the present invention is to realize a smaller size, lower power consumption, and a longer service life in an NDIR optical gas sensor device.

### Solution to Problem

To achieve the abovementioned object, the optical gas sensor device according to the present invention includes:
a substrate;
a light source that is mounted on a plane of the substrate, that includes a light emitting surface facing the same direction as the plane of the substrate, and that emits infrared rays from the light emitting surface;
a filter that transmits infrared rays with a wavelength corresponding to an absorption wavelength of a gas as a detection target among the infrared rays and emits the transmitted infrared rays to the gas as the detection target;
a light receiver that detects the infrared rays incident through the gas as the detection target;
a cover which is provided on the substrate so as to cover the light source and the light receiver, that reflects the infrared rays passing the filter on an inner surface of the cover, and that is provided so that at least some of the reflected light reaches the light receiver; and
a gas introducer that introduces gas as the detection target into the cover.

### Advantageous Effects of Invention

According to the present invention, it is possible to realize a smaller size, lower power consumption, and a longer service life in an NDIR optical gas sensor device.

### BRIEF DESCRIPTION OF DRAWINGS

[FIG. 1] This is a schematic diagram of an optical gas sensor device according to an embodiment of the present invention.
[FIG. 2] This is a diagram showing absorption wavelengths of multiple types of gases.
[FIG. 3] This is a partially transparent perspective view of the optical gas sensor device according to the present embodiment.
[FIG. 4] This is a partially transparent plan view of the optical gas sensor device according to the present embodiment.
[FIG. 5] This is a perspective view of a light source.
[FIG. 6] This is a cross-sectional view of the light source and an optical filter.
[FIG. 7A] This is a schematic plan view showing an optical path of the optical gas sensor device according to the present embodiment.
[FIG. 7B] This is a schematic side view showing the optical path of the optical gas sensor device according to the present embodiment.
[FIG. 8A] This is a schematic plan view showing an optical path of the optical gas sensor device according to a modification.
[FIG. 8B] This is a schematic side view showing the optical path of the optical gas sensor device according to the modification.

### DESCRIPTION OF EMBODIMENTS

Hereinafter, embodiments and modifications of the present invention will be described in detail in order with reference to the accompanying drawings. However, the scope of the present invention is not limited to the embodiments or illustrated examples.

### (Embodiment)

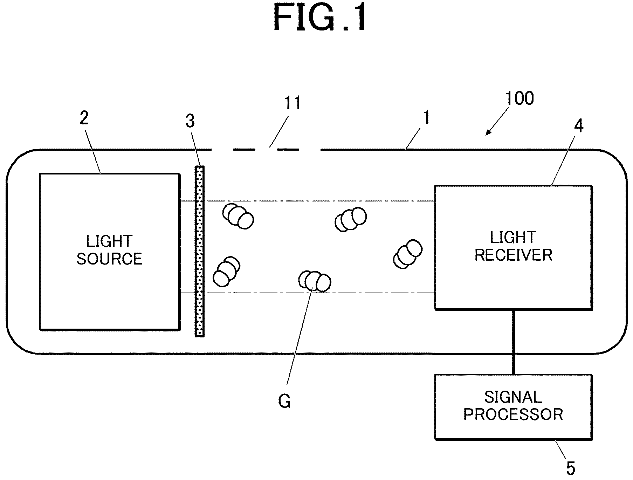

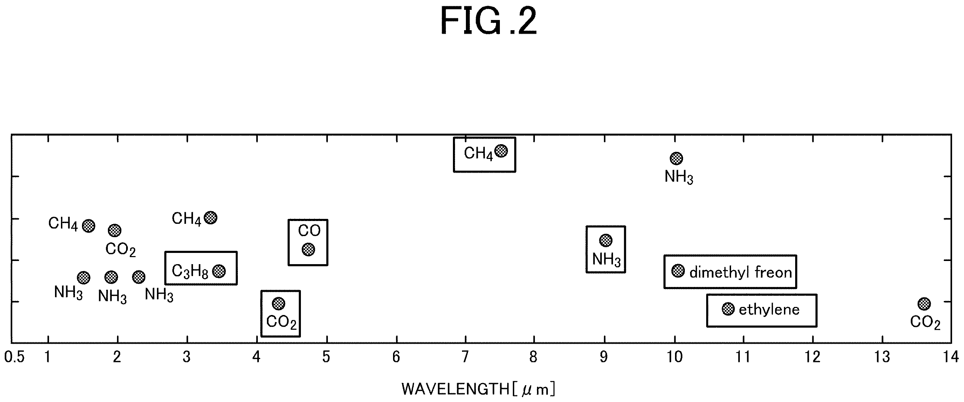

Embodiments according to the present invention will be described with reference to FIGS. 1 to 7B. First, a schematic configuration of an optical gas sensor device 100 according to the present embodiment will be described with reference to FIGS. 1 and 2. FIG. 1 is a schematic diagram of the optical gas sensor device 100 according to the present embodiment. FIG. 2 is a diagram showing absorption wavelengths of multiple types of gases.

As shown in FIG. 1, the optical gas sensor device 100 according to the present embodiment includes a cover 1, a light source 2 as a light source section, an optical filter 3 as a filter, a light receiver 4, and a signal processor 5. The optical gas sensor device 100 is an NDIR gas sensor. The optical gas sensor device 100 emits infrared rays (emits light) from the light source 2, and emits the infrared rays to a gas G as a detection target in the cover 1 through an optical path in the cover 1. The molecules of the gas G as the detection target present in the optical path absorb the infrared rays and the amount of light reaching the light receiver 4 is reduced. The light receiver 4 detects the infrared rays partially absorbed by the gas G as the detection target. A detection signal is sent to the signal processor 5. The signal processor 5 processes the detection signal and outputs the concentration of the gas G as the detection target. The cover 1 has a gas introduction port 11 as a gas introduction section that is an inlet/outlet for the gas G as the detection target. Specifically, the optical gas sensor device 100 filters infrared rays emitted from the light source 2 with the optical filter 3 and emits the infrared rays to the gas G as the detection target.

CO₂ is to be used as the gas G which is the detection target. FIG. 2 shows the wavelengths (absorption wavelengths) [µm] of light absorbed by multiple types of gases. In FIG. 2, (the molecule of) the same type of gas is shown, and this indicates that the same type of gas has a plurality of absorption wavelengths. Furthermore, in FIG. 2, a quadrilateral box is attached to a molecule of a gas having the largest absorption wavelength among a plurality of absorption wavelengths of the same type of gas. CO₂ also has a plurality of absorption wavelengths, and the optical gas sensor device 100 detects the absorption of infrared rays at a wavelength of 4.26 [pm], which has the largest absorption among the absorption wavelengths of CO₂.

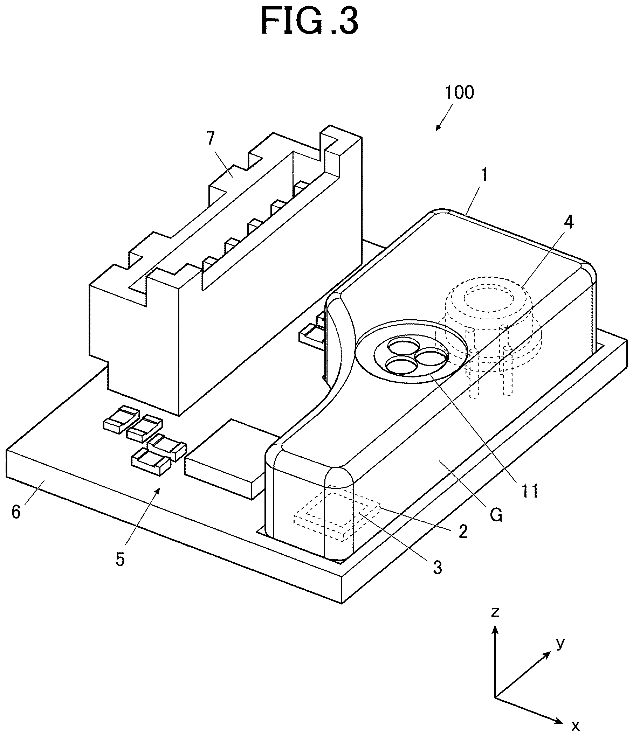

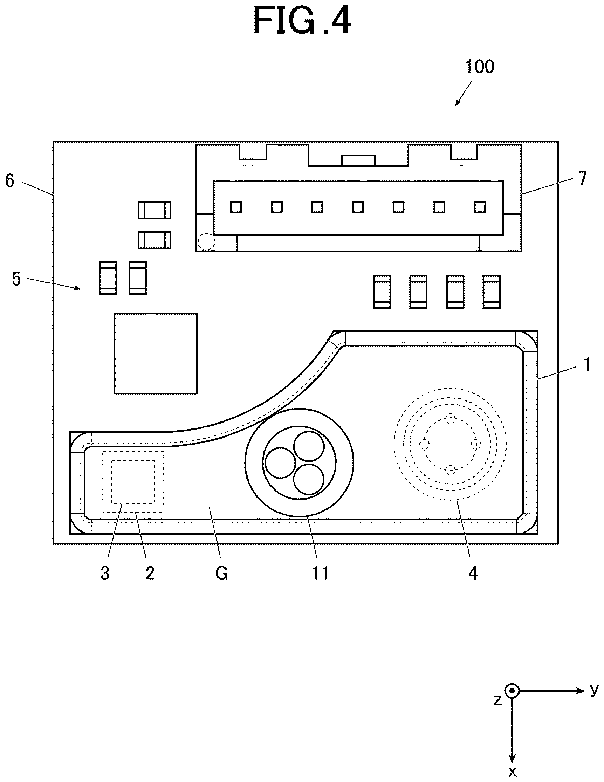

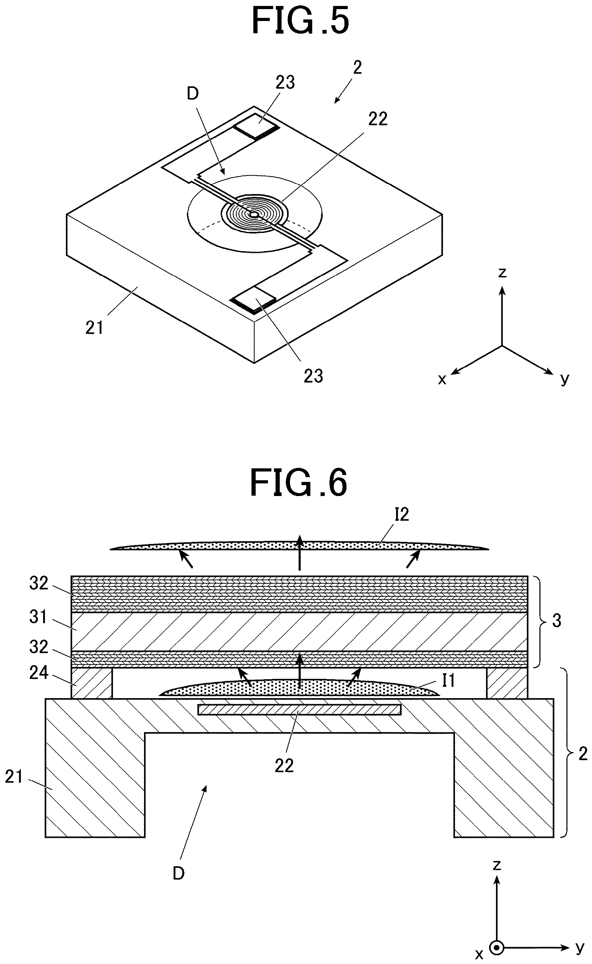

Next, a specific configuration of the optical gas sensor device 100 will be described with reference to FIGS. 3 to 6. FIG. 3 is a partially transparent perspective view of the optical gas sensor device 100 according to the present embodiment. FIG. 4 is a partially transparent plan view of the optical gas sensor device 100 according to the present embodiment. FIG. 5 is a perspective view of the light source 2. FIG. 6 is a cross-sectional view of the light source 2 and the optical filter 3.

As shown in FIGS. 3 and 4, specifically, the optical gas sensor device 100 includes the cover 1, the light source 2, the optical filter 3, the light receiver 4, the signal processor 5, a substrate 6, and a connector 7. Further, in FIGS. 3 and 4, the x-axis, y-axis, and z-axis are illustrated. These three axes are the same in FIGS. 5 to 8B.

The cover 1 is a cover with the following features. The cover 1 is mounted on the +z side plane (xy plane) of the substrate 6. The cover 1 covers (includes) the light source 2, the optical filter 3, and the light receiver 4 and forms a space therein that can accommodate the gas G as the detection target. The cover 1 is provided so that the infrared rays that have passed through the optical filter 3 are reflected on the inner surface of the cover 1 and at least some of the reflected infrared light reaches the light receiver 4. This gas G as the detection target is introduced in and out of the space through the gas introduction port 11. A base of the cover 1 is made of resin, for example, and has a plurality of flat or curved inner surfaces. The inner surface of the base of the cover 1 is covered with an infrared reflective film. In this embodiment, gold is used as the infrared reflective film, but the material is not limited to this. Silver, aluminum, or a dielectric multilayer film may be used as the infrared reflective film. Further, as necessary, a protective film such as silicon oxide or silicon nitride may be formed on the infrared reflective film in order to prevent corrosion of a metal film of the infrared reflective film. As a method for forming the infrared reflective film and the protective film, a plating method, a sputtering method, a vacuum evaporation method, etc. can be used.

The cover 1 plays a role as the optical path to efficiently guide the infrared rays from the light source 2 to the light receiver 4 by reflecting the infrared rays emitted from the light source 2 with an infrared reflecting film. The light path of the infrared rays in the optical gas sensor device 100 will be described later.

The gas introduction port 11 is provided on the cover 1, includes three holes, and introduces and outputs the gas G as the detection target into and out of the space inside the cover 1. Note that the shape, size, and position of the gas introduction port 11 on the cover 1 shown in FIGS. 3 and 4 are merely examples, and the features are not limited to the above. According to the present embodiment, the gas introduction port 11 as the gas introduction section is provided in the cover 1, but the present invention is not limited to this. For example, a configuration in which the gas introduction section is provided on the substrate 6 may be employed (for example, a configuration in which the gas introduction section is a hole opened at a position on the substrate 6 that communicates with the space inside the cover 1, and the gas G as the detection target is introduced into and emitted out of the space inside the cover 1.)

The light source 2 is mounted on the +z side plane of the substrate 6, includes a light emitting surface facing the same direction (+z direction) as the +z side plane of the substrate 6, and emits infrared rays from the light emitting surface. Further, the light source 2 is a MEMS (Micro Electro Mechanical Systems) type light source, and includes, for example, a diaphragm structure. As shown in FIGS. 5 and 6, the light source 2 includes a silicon chip 21 as a base section, a thin film heater 22, a wire bonding pad 23, and a bonding portion 24. The silicon chip 21 is a semiconductor chip mainly made of silicon, and includes a diaphragm D made of a laminated film of silicon oxide and silicon nitride in the center of the plane (xy plane) of the silicon chip 21.

The thin film heater 22 is a light source that emits infrared rays, and is formed substantially at the center of the plane of the diaphragm D. The thin film heater 22 is connected to an extraction electrode via a contact portion on the diaphragm D or around the silicon chip 21, and is electrically connected to the wire bonding pad 23. The materials that can be used as the thin film heater 22 include high melting point metals such as tungsten (melting point: 3387 [°C]), rhenium (melting point 3180 [°C]), tantalum (melting point 2996 [°C]), osmium (melting point 2700 [°C]), molybdenum (melting point 2610 [°C]), niobium (melting point 2468 [°C]), iridium (melting point 2447 [°C]), boron (melting point 2300 [°C]), ruthenium (melting point 2250 [°C]), or hafnium (melting point 2150 [°C]), impurity-doped silicon, or conductive oxide. The diaphragm D of the thin film heater 22 is heated by energization, and emits infrared rays having a strength and wavelength ionicity depending on surface temperature and surface emissivity.

The thin film heater 22 is patterned on the silicon chip 21 by, for example, lithography. According to the present embodiment, the thin film heater 22 is directly mounted on the substrate 6 (COB: Chip On Board). However, the present embodiment is not limited to this, and the thin film heater 22 may be housed in a CAN package, a ceramic package, or the like. The thin film heater 22 is not limited to a circular shape, but may be polygonal, and has a size that does not exceed the region of the diaphragm D.

The wire bonding pad 23 is wire-bonded to wiring on the substrate 6. The bonding portion 24 is a bonding portion that is disposed on the silicon chip 21 and that bonds the optical filter 3 to the silicon chip 21. As a method for bonding the bonding portion 24, metal bonding, glass bonding, anodic bonding, solder bonding, resin adhesion, etc. can be used. Since the thin film heater 22 is formed on the diaphragm D, the heat capacity of the light source 2 can be lowered and the thermal efficiency can be increased.

The optical filter 3 is a filter provided in a planar shape directly above (+z side) and facing the thin film heater 22 via the bonding portion 24, and transmits light (infrared rays) with a wavelength range (band) corresponding to the absorption wavelength specific to the gas G as the detection target. As described above, the transmission wavelength of the optical filter 3 is designed to match the unique absorption wavelength of the gas G as the detection target. With this, changes in the amount of light due to gases other than the gas G as the detection target is suppressed, and the signal to noise ratio (SN ratio) of the detection signal of the light receiver 4 is improved. More specifically, as shown in FIG. 6, the optical filter 3 filters the infrared rays I1 having a wide wavelength range incident from the light source 2, and transmits infrared rays I2 in a wavelength range corresponding to an absorption wavelength (4.26 um) of the gas G (CO₂).

The optical filter 3 includes, for example, a silicon substrate 31 as a semiconductor substrate and a dielectric multilayer film 32. The silicon substrate 31 is a silicon substrate provided in a planar shape directly above and facing the thin film heater 22. The dielectric multilayer film 32 is a film in the form of a plurality of dielectric layers provided on both sides of the silicon substrate 31. Although the dielectric multilayer film 32 is provided on both sides of the silicon substrate 31, it is not necessary that the dielectric multilayer films 32 on both sides have an equivalent film structure, and they may have different film structures. Alternatively, the dielectric multilayer film 32 may be provided only on one side of the silicon substrate 31. Although the planar shape of the optical filter 3 is rectangular, the shape is not limited to this, and the shape may be other shapes such as a circle.

The light source 2 as a MEMS type light source is small and low in height, and can realize a smaller size as a sensor module, especially reducing the height compared to a conventional incandescent light source or an LED (Light Emitting Diode). Conventional incandescent light sources have problems such as large deterioration over time, high current consumption, large variations in light source position, limited emission wavelength bands (up to 5 [pm]), long response time, large size, and the like. Conventional LEDs have problems such as low amount of light, large temperature characteristics, and high cost.

On the other hand, the light source 2 as the MEMS type light source has characteristics (features) such as long life, low power consumption, and short response time. By reducing the power consumption of the light source which is the main component of the sensor module, the reduction of power consumption of the sensor module can be achieved. The short response time of the MEMS light source makes it possible to shorten the standby time after energization when intermittent driving is performed, thereby reducing average power consumption.

Furthermore, since the infrared rays obtained from conventional incandescent light sources are emitted through a glass spherical shell, the strength of the infrared rays on a higher wavelength side decreases due to absorption by the glass. On the other hand, the light source 2 as a MEMS type light source can directly utilize the emitted light from the surface of a high-temperature part, so it can also be applied to the detection of gases having absorption bands at high wavelengths. The infrared ray emitting region (thin film heater 22) of the light source 2 is patterned with high precision on the plane of the silicon chip 21, and unlike a conventional incandescent light source in which a filament is wound into a coil, the individual variation of the emission direction is very small. Therefore, variations in the amount of light received when a sensor module is configured with the light source 2 are reduced, and this contributes to an improvement in product yield.

Furthermore, since the light source 2 has a flat light emitting surface, the optical filter 3, which also has a plate-like flat surface, can be easily mounted in a space-saving manner.

In addition, the light source 2 and the optical filter 3 are produced in bulk using MEMS technology based on silicon wafers, so these are excellent in mass productivity. For example, as a method for manufacturing the light source 2 and the optical filter 3, there is a method in which a plurality of thin film heaters 22, wire bonding pads 23, bonding portions 24, and the optical filter 3 are formed on one silicon wafer, and the above is diced and formed into chips to be the individual light source 2 and the optical filter 3. Alternatively, as a manufacturing method of the light source 2 and the optical filter 3, a plurality of silicon chips 21 may be manufactured by dicing one silicon wafer, and the thin film heater 22, the wire bonding pad 23, the bonding portions 24, and the optical filter 3 may be formed on each silicon chip 21. In this way, the light source 2 and the optical filter 3 can be fabricated using the chipping process and mounting method as in the manufacture of semiconductors and MEMS devices, so the productivity of the mounting process is high. Although the optical filter 3 is illustrated as having a uniform thickness, the infrared light transmitting region on the plane may be thinner than other regions. This makes it possible to suppress a decrease in the strength of the emitted light due to absorption by the silicon substrate 31 (Si base material), and also to improve the emission efficiency of the light source by reducing the heat capacity.

The light receiver 4 is a thermopile-type infrared sensor that is mounted on a +z side plane of the substrate 6 and that includes a plurality of thermocouples, and detects the amount of incident infrared light and outputs a detection signal as an analog electrical signal. However, the light receiver 4 is not limited to the thermopile type infrared sensor, and may be an infrared sensor using a photodiode, a bolometer, a pyroelectric sensor, or the like. Further, the light receiver 4 is, for example, an infrared sensor of a CAN package, but the configuration is not limited to this.

The signal processor 5 is a circuit that is mounted on a plane area other than the cover 1 on the +z side plane of the substrate 6 and processes the detection signal of the light receiver 4. The signal processor 5 includes an AFE (Analog Front End)-IC (Integrated Circuit), a chip resistor, a chip capacitor, and the like. The AFE-IC includes an amplifier circuit, an AD (Analog to Digital) conversion circuit, and the like. The signal processor 5 amplifies an analog detection signal from the light receiver 4 and performs AD conversion. The signal processor 5 performs signal processing such as correction of individual variations in the optical gas sensor device 100, and outputs a digital detection signal. The signal processor 5 may include components such as a voltage regulator, a preamplifier, and a transistor element as necessary.

The substrate 6 is a PCB (Printed Circuit Board) in which conductor wiring is printed on a board made of glass epoxy resin or the like. On the +z side plane of the substrate 6, the cover 1, the light source 2 (and the optical filter 3), the light receiver 4, the signal processor 5, and the connector 7 are mounted. In this configuration, all the components are mounted on one substrate 6, but the optical components may be separate components mounted in advance on another substrate.

The connector 7 is a connector mounted on a plane region other than the cover 1 and the signal processor 5 on the +z side surface of the substrate 6 and outputs the digital detection signal output from the signal processor 5. The connector 7 is connected to an electronic device via a cable with a plug. This electronic device outputs gas concentration as an analog voltage or a digital value such as I2C (Inter-Integrated Circuit), SPI (Serial Peripheral Interface), or UART (Universal Asynchronous Receiver/Transmitter). Alternatively, it is an alarm device that outputs an alarm when the concentration of the gas G detected by the optical gas sensor device 100 becomes equal to or more than a predetermined threshold value. Note that the optical gas sensor device 100 may be configured to be provided within the electronic device.

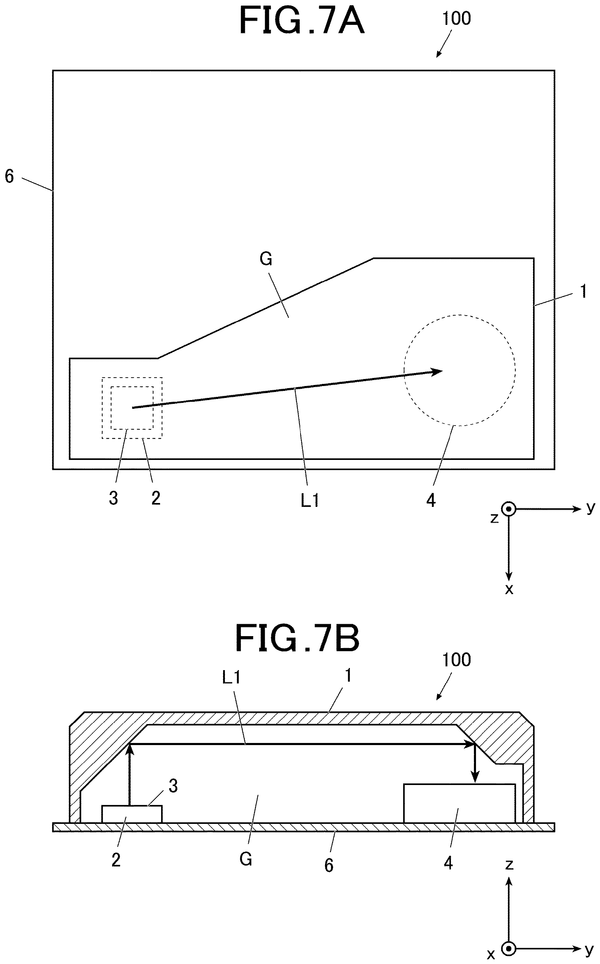

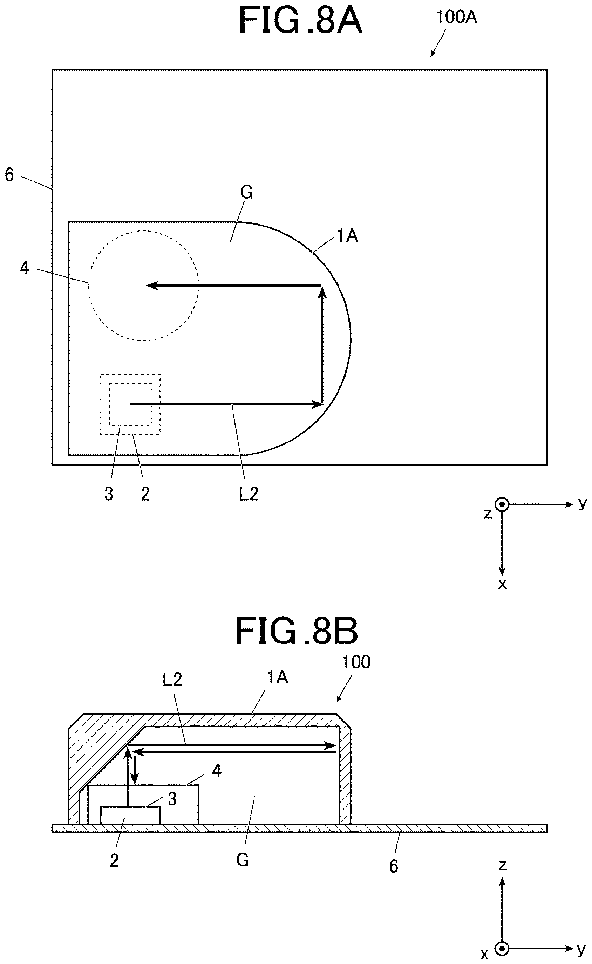

Next, the optical path of infrared rays of the optical gas sensor device 100 will be explained with reference to FIGS. 7A and 7B. FIG. 7A is a schematic plan view showing an optical path L1 of the optical gas sensor device 100 according to the present embodiment. FIG. 7B is a schematic side view showing the optical path L1 of the optical gas sensor device 100 according to the present embodiment. Note that in FIGS. 7A and 7B, illustrations of parts of the optical gas sensor device 100 that are not necessary for explaining the optical path are appropriately omitted, and the same applies to FIGS. 8A and 8B described later.

As shown in FIGS. 7A and 7B, in the optical gas sensor device 100, infrared rays emitted from the light source 2 in the +z direction are filtered by the optical filter 3, absorbed by the gas G as the target to be measured in the cover 1, reflected in the +y direction by the planar inner surface of the cover 1, further reflected in the -z direction by the planar inner surface of the cover 1, and passes through an optical path L1 entering the light receiver 4. The optical path L1 is a linear optical path on the plane. In other words, the inner surface of the cover 1 is formed in a shape corresponding to the optical path L1. Since the direction of the light emitted from the light source 2 has a distribution, there is a considerable number of light components that reach the light receiver 4 through multiple reflections, not limited to the linear optical path L1.

As described above, according to the present embodiment, the optical gas sensor device 100 includes the substrate 6, the light source 2, the optical filter 3, the light receiver 4, the cover 1, and the gas introduction port 11. The light source 2 is mounted on the plane of the substrate 6, has a light emitting surface facing in the same direction as the plane of the substrate 6, and emits the infrared rays from the light emitting surface. The optical filter 3 transmits infrared rays of a wavelength corresponding to the absorption wavelength of the gas G as the detection target among the infrared rays and emits the infrared rays to the gas G as the detection target. The light receiver 4 detects incident infrared rays through the gas G as the detection target. The cover 1 is provided on the substrate 6 so as to cover the light source 2 and the light receiver 4. The cover 1 is provided so that the infrared rays that have passed through the optical filter 3 are reflected on the inner surface of the cover 1 and at least some of the reflected light reaches the light receiver 4. The gas introduction port 11 introduces the gas G as the detection target into the inside of the cover 1. Therefore, it is possible to realize a smaller size, lower power consumption, and a longer service life in the NDIR optical gas sensor device 100.

Further, the optical filter 3 includes the dielectric multilayer film 32 formed on the silicon substrate 31. Therefore, infrared rays having a wavelength corresponding to the absorption wavelength of the gas G as the detection target can be transmitted easily and accurately.

Further, the light source 2 is a MEMS type light source. More specifically, the light source 2 includes the silicon chip 21 having the diaphragm D, and the thin film heater 22 formed on the diaphragm D. Therefore, since the light source 2 is small and low in height, the optical gas sensor device 100 as the sensor module can be made small and low in height. In addition, with the light source 2, compared to conventional light sources, power consumption can be reduced, the average power consumption can be reduced because the response time can be made shorter. Moreover, since there is no glass, the strength of the infrared rays can be increased, and the gas G with the absorption wavelength of high wavelengths can be detected. Further, individual variations of the direction of infrared emission is reduced, thereby improving product yield and extending service life. The MEMS technology can increase the mass productivity of the light source 2 and increase the productivity of the mounting process. Since the light source 2 is planar, the optical filter 3 can be easily mounted in a space-saving manner. Since the light source 2 has the diaphragm structure, the heat capacity can be lowered and the thermal efficiency can be increased.

Further, the cover 1 forms a space in which the gas G as the detection target can be stored. Moreover, the cover 1 forms the optical path L1 of infrared rays passing through the gas G as the detection target by the planar inner surface. Therefore, the gas G as the detection target can be detected easily and accurately.

The infrared reflective film that reflects infrared rays is included on the inner surface of the cover 1. Therefore, the gas G as the detection target can be detected more accurately.

Further, the gas introduction port 11 is provided in the cover 1. Therefore, the gas introduction port 11 can be easily configured, and the gas G as the detection target can be effectively introduced and output.

Further, the light receiver 4 includes the infrared sensor using one of a thermopile type, a photodiode, a bolometer, and a pyroelectric sensor. Therefore, the light receiver 4 can be made easily and the gas G as the detection target can be detected accurately.

### (Modification)

A modification of the above embodiment will be described with reference to FIGS. 8A and 8B. FIG. 8A is a schematic plan view showing an optical path L2 of an optical gas sensor device 100A according to the modification. FIG. 8B is a schematic side view showing the optical path L2 of the optical gas sensor device 100A according to the modification.

As shown in FIGS. 8A and 8B, an optical gas sensor device 100A is used as a device configuration of this modification. The optical gas sensor device 100A includes a configuration in which the cover 1 of the optical gas sensor device 100 according to the above embodiment is replaced with a cover 1A. Therefore, in the optical gas sensor device 100A, the same parts as those in the optical gas sensor device 100 are given the same reference numerals, and the explanation thereof is omitted.

Similar to the cover 1, the cover 1A is mounted on the +z side surface of the substrate 6 and includes a light source 2, an optical filter 3, and a light receiver 4, but its shape, especially the shape of the inner surface, may be a flat or curved surface. The light source 2, the optical filter 3, and the light receiver 4 are mounted at different positions on the +z side surface of the substrate 6 from those of the optical gas sensor device 100.

As shown in FIGS. 8A and 8B, in the optical gas sensor device 100A, infrared rays emitted from the light source 2 in the +z direction are filtered by the optical filter 3, absorbed by the gas G in the cover 1A, reflected in the +y direction by the planar inner surface of the cover 1A, further reflected two times in the -x direction to the -y direction on the curved inner surface of the cover 1A, further reflected in the -z direction by the planar inner surface of the cover 1A, and passes through an optical path L2 entering the light receiver 4. The optical path L2 is a returning optical path on the plane. In other words, the inner surface of the cover 1A is formed in a shape corresponding to the optical path L2. Since the direction of the light emitted from the light source 2 has a distribution, there is a considerable number of light components that reach the light receiver 4 through multiple reflections, not limited to the linear optical path.

As described above, according to this modification, the optical gas sensor device 100A includes the cover 1A. The cover 1A forms the optical path L2 of infrared rays passing through the gas G as the detection target by the planar inner surface and the curved inner surface. Therefore, the gas G as the detection target can be detected easily and accurately, and the degree of freedom in arranging the light source 2 (and optical filter 3) and the light receiver 4 can be increased. Furthermore, since the optical path L2 is common in both directions, the physical length of the optical path L2 can be shortened, and the sensor module (optical gas sensor device 100A) can be made smaller.

The description of the above-described embodiments and modification are examples of the optical gas sensor device according to the present invention, but the present invention is not limited to the above. For example, the configuration of the above embodiment and the configuration of the modification may be combined as appropriate.

For example, in the above embodiments and modifications, the gas G as the target to be detected by the optical gas sensor devices 100, 100A is CO₂, but the present invention is not limited to this. Other gas molecules that absorb infrared rays can be selected as the gas G as the detection target. Other gas molecules of the gas G as the detection target include, for example, each of the gas molecules shown in FIG. 2, such as propane (C₃H₈), carbon monoxide (CO), methane (CH₄), ammonia (NH₃), ethylene (C₂H₄), and dimethylfuran (in FIG. 2, the absorption wavelength surrounded with a quadrilateral is the absorption wavelength with the largest absorption among the absorption wavelengths of the gas molecules).

According to the above embodiments and modifications, the optical gas sensor devices 100, 100A include one set of a set including the light source 2, the optical filter 3, and the light receiver 4, but the present invention is not limited to this. The optical gas sensor device may include a plurality of sets of light sources 2, optical filters 3, and light receivers 4.

According to the above embodiments and modifications, the optical filter 3 is fixed to the light source 2, but the present invention is not limited to this. For example, a configuration may be adopted in which the optical filters 3 corresponding to different types of gases G as the detection target are detachably attached to the light source 2.

In addition, the detailed configuration and detailed operation of the optical gas sensor device in the above-described embodiments and modifications can be changed as appropriate without departing from the spirit of the present invention.

### Industrial Applicability

As described above, the optical gas sensor device according to the present invention is suitable for detecting gases such as CO₂.

### Reference Signs List

100, 100A optical gas sensor device
G gas
1, 1A Cover
11 Gas introduction port
2 Light source
21 Silicon chip
22 Thin film heater
23 Wire bonding pad
24 Bonding portion
3 Optical filter
31 Silicon substrate
32 Dielectric multilayer film
4 Light receiver
5 Signal processor
6 Substrate
7 Connector

## Claims

1. An optical gas sensor device comprising:
a substrate;
a light source that is mounted on a plane of the substrate, that includes a light emitting surface facing the same direction as the plane of the substrate, and that emits infrared rays from the light emitting surface;
a filter that transmits infrared rays with a wavelength corresponding to an absorption wavelength of a gas as a detection target among the infrared rays and emits the transmitted infrared rays to the gas as the detection target;
a light receiver that detects the infrared rays incident through the gas as the detection target;
a cover which is provided on the substrate so as to cover the light source and the light receiver, that reflects the infrared rays passing the filter on an inner surface of the cover, and that is provided so that at least some of the reflected light reaches the light receiver; and
a gas introducer that introduces gas as the detection target into the cover.

2. The optical gas sensor device according to claim 1, wherein the filter includes a dielectric multilayer film formed on a semiconductor substrate.

3. The optical gas sensor device according to claim 1 or 2, wherein the light source is a MEMS type light source.

4. The optical gas sensor device according to claim 3, wherein,
the light source is formed from a semiconductor substrate; and
the light source includes,
a base including a diaphragm, and
a thin film heater formed on the diaphragm.

5. The optical gas sensor device according to any one of claims 1 to 4, wherein the cover forms a space capable of storing the gas as the detection target.

6. The optical gas sensor device according to any one of claims 1 to 5, wherein the cover forms an optical path of the infrared rays passing through the gas as the detection target by at least one of a planar inner surface and a curved inner surface.

7. The optical gas sensor device according to any one of claims 1 to 6, wherein the cover includes on its inner surface an infrared reflective film that reflects infrared rays.

8. The optical gas sensor device according to any one of claims 1 to 7, wherein the gas introducer is provided in the cover.

9. The optical gas sensor device according to any one of claims 1 to 8, wherein the light receiver includes an infrared sensor using one of a thermopile type, a photodiode, a bolometer, and a pyroelectric sensor.
